# FASCICULE DE BREVET EUROPEEN

(11) **EP 2 635 302 B1**
(45) Date de publication et mention de la délivrance du brevet: **24.05.2017**
(21) Numéro de dépôt: 11802469.4
(22) Date de dépôt: 04.11.2011
(51) Int. Cl.: A61K 39/145, C12N 7/02

(54) **PROCEDE POUR AMELIORER LA PRODUCTION DE VIRUS ET SEMENCES VACCINALES INFLUENZA**
VERFAHREN ZUR VERBESSERUNG DER HERSTELLUNG VON INFLUENZAVIREN UND IMPFKRISTALLEN
METHOD FOR IMPROVING THE PRODUCTION OF INFLUENZA VIRUSES AND VACCINE SEEDS

(30) Priorité: 05.11.2010 FR 1059132
(43) Date de publication de la demande: 11.09.2013
(73) Titulaire: University of Dundee, Dundee DD1 4HN (GB); Centre National de la Recherche Scientifique, 75794 Paris Cedex 16 (FR); Université Claude Bernard Lyon I, 69622 Villeurbanne Cedex (FR); Hospices Civils de Lyon, 69229 Lyon Cedex 02 (FR)
(72) Inventeur: TERRIER, Olivier, St Andrews KY169UG (GB); BOURDON, Jean-Christophe, Dundee DD21QD (GB); ROSA-CALATRAVA, Manuel, 69003 Lyon (FR)
(74) Mandataire: Sarlin, Laure V.
(86) Numéro de dépôt international: PCT/FR2011/052575
(87) Numéro de publication internationale: WO 2012/059696

(56) Documents cités:
- EP-A1- 1 739 167
- WO-A2-2008/130614
- US-A1- 2004 142 450
- DATABASE CA [Online] CHEMICAL ABSTRACTS SERVICE, COLUMBUS, OHIO, US; 19 mars 2009 (2009-03-19), DUAN, MING ET AL: "Influences on MDM2 and p53 in influenza virus-induced apoptosis in human A549 cells", XP002635306, extrait de STN Database accession no. 2009:330316 & ZHONGGUO SHOUYI XUEBAO , 29(1), 12-14, 24 CODEN: ZSXUF5; ISSN: 1005-4545, 2009,
- CARVAJAL D ET AL: "activation of p53 by MDM2 antagonists can protect proliferating cells from mitotic inhibitors", CANCER RESEARCH, AMERICAN ASSOCIATION FOR CANCER RESEARCH, US, vol. 65, no. 5, 1 March 2005 (2005-03-01), pages 1918-1924, XP002338501, ISSN: 0008-5472, DOI: 10.1158/0008-5472.CAN-04-3576

## Description

La présente invention concerne le domaine technique des virus et des semences vaccinales influenza. Plus précisément, l'invention concerne des procédés pour améliorer la production des virus influenza et des semences vaccinales influenza.

La grippe est une infection respiratoire virale fréquente, observée partout dans le monde, évoluant par bouffées épidémiques hivernales dans les régions tempérées, due aux virus influenza. Elle reste de nos jours la deuxième cause de mortalité infectieuse après les pneumonies. Les virus influenza responsables de pathologies chez l'homme sont les virus influenza de type A et B. Alors que les virus influenza de type B circulent sous forme de lignage, les virus influenza de type A sont classés en sous types viraux en fonction des propriétés antigéniques des deux glycoprotéines majeures de surface, l'hémagglutinine (HA) et la neuraminidase (NA). Les virus influenza présentent entre 300 et 700 glycoprotéines à leur surface associée à un ratio théorique NA/HA de un pour dix. Les virus circulants chez l'homme et responsables des épidémies saisonnières sont les virus A (H1N1) et A (H3N2). Le principal réservoir des virus influenza étant le réservoir animal (aviaire et porcin), des virus animaux peuvent franchir la barrière d'espèce et infecter l'homme. Des virus comme le virus aviaire hautement pathogène A (H5N1) et le virus A (H1N1) responsable de la pandémie de 2009 peuvent entrainer de graves problèmes de santé publique.

La vaccination est, pour l'instant, le seul moyen efficace pour protéger les populations des virus influenza. Le vaccin dit saisonnier permet d'acquérir une immunité contre les virus circulants saisonniers A (H1N1), A (H3N2) et les virus B. Il est défini chaque année par l'OMS à partir des souches prototypes de l'année antérieure. La réponse immunitaire de l'hôte est principalement de type humorale avec synthèse d'anticorps neutralisants qui sont dirigés contre les protéines HA et NA. En raison d'une dérive antigénique importante de ces deux protéines, en particulier pour les virus de type A, la composition vaccinale doit être réévaluée annuellement.

La culture des virus influenza constitue un élément critique dans le domaine de la production de vaccins, mais également dans le domaine de la recherche fondamentale et biomédicale sur les virus influenza. Les virus influenza réassortant vaccinaux sont principalement cultivés dans le système d'oeufs de poule embryonnés. On estime actuellement qu'un oeuf permet la production d'une dose de vaccin trivalent (Hampson et al. Influenza Other Respi Viruses. 2008 Nov. 2(6), 191-2). Le procédé de production de vaccin sur oeufs nécessite une échelle de temps incompressible de 5 à 6 mois. Pour faire face à la demande croissante de vaccins contre les souches circulantes saisonnières, mais également à la demande - difficilement prévisible - de vaccins contre une (ou plusieurs) souches émergentes potentiellement pandémiques, la disponibilité des oeufs peut s'avérer être un facteur limitant, d'autant plus que des risques de pandémies aviaires dans les élevages persistent. En outre, les volailles elles-mêmes sont susceptibles d'être affectés par des virus influenza ce qui pourrait causer des difficultés d'approvisionnement si des élevages destinés à la production de virus étaient eux-mêmes concernés par une pandémie de virus influenza. Dans ce contexte et d'un point de vue économique, la recherche et la mise au point de nouveaux procédés optimisés de production des semences vaccinales sont légitimes (réduction de temps et/ou réduction des coûts).

Des stratégies alternatives pour l'obtention des doses vaccinales se sont développées ces dernières années. En effet, l'utilisation de lignées cellulaires pour l'amplification des réassortants vaccinaux permet entre autre de ne plus être dépendant du système «oeuf» (quantité d'ouf potentiellement insuffisante pour la gestion d'une pandémie), réduit les modifications des antigènes de surface régulièrement observées dans la production allantoïque et entrainerait moins de risques d'allergies. Cependant, à l'heure actuelle, peu d'industriels ont choisi ce nouveau mode de production car le procédé industriel est loin d'être aussi performant que celui en système allantoïque. En effet, tant la production de virus que la production d'antigènes viraux (HA et NA) en culture cellulaire se heurtent à des difficultés en termes de rendement et donc de quantité de virus ou d'antigènes produits. De nombreuses équipes de recherche développent actuellement des systèmes cellulaires de production de virus, pour compléter voire substituer le modèle de production sur oeuf. Ces systèmes cellulaires sont généralement permissifs pour un nombre plus large de souches virales que dans le système allantoïque et peuvent être rapidement mis en place à l'échelle de l'industrialisation (Barrett et al. Curr Opin Mol Ther. 2010 Feb;12(1):21-30). De plus, ces systèmes peuvent être couplés avec les techniques de génétique inverse, qui permettent de produire des virus recombinants (semences vaccinales) de manière rapide et flexible, mais également optimisés pour leur propriétés virologiques (réplication, expression d'antigène...etc). Pour des raisons de sécurité sanitaire et réglementaires, les lignées cellulaires choisies doivent être sélectionnées pour leur capacité à produire du virus avec des hauts titres, dans des milieux synthétiques dépourvus de protéines d'origine animale (milieux synthétiques sans sérum d'origine animal). Des systèmes cellulaires de production en cellules en suspension ont également été décrits (Le Ru et al. Vaccine. 2010 May 7;28(21):3661-71). Les lignées cellulaires bénéficiant d'autorisations réglementaires telles que les lignées MDCK, Vero, BHK21, CHO, HEK 293 et PERC6 n'ont pas permis, jusqu'à ce jour, d'obtenir des rendements de production adéquats pour les industriels.

Un des enjeux économiques majeur est de pouvoir réduire le coût et les délais de fabrication d'une dose vaccinale (plus de doses par production et/ou réduction du temps d'obtention de la même quantité de doses).

L'invention repose sur l'observation inattendue qu'une action antagoniste à l'activité de Mdm2 et plus particulièrement inhibitrice de l'interaction p53-Mdm2 a un impact sur le cycle réplicatif des virus influenza.

Dans ce contexte, la présente invention propose un nouveau procédé de production des virus et semences vaccinales influenza caractérisé en ce que la production est réalisée *in vitro* en culture cellulaire, dans une lignée cellulaire MDCK, en présence d'un antagoniste de la protéine Mdm2 tel que défini à la revendication 1.

L'antagoniste de la protéine Mdm2 est en particulier utilisé pour améliorer quantitativement la production du virus influenza produit, ce qui n'était pas connu ni suggéré dans l'art antérieur.

Selon l'invention, l'antagoniste de la protéine Mdm2 est un inhibiteur de l'interaction entre la protéine Mdm2 et la protéine p53.

Avant de décrire de manière plus détaillée l'invention, certaines définitions des termes utilisés dans le cadre de l'invention vont être données.

Par « virus influenza », on entend désigner tous les virus influenza, et notamment les virus influenza humains, aviaires, équins, porcins et félins. Lesdits virus influenza peuvent être sélectionnés parmi les sous-types A, B et C. En particulier, le virus influenza peut être de sous-type A et notamment correspondre aux souches H1N1, H2N2, H3N2, H4N2, H4N6, H5N1, H5N2, H7N7 et H9N2, et en particulier les virus A (H1N1) et A (H3N2). Parmi les souches H1N1, on peut plus particulièrement citer: A/Porto Rico/8/34 (également nommée A/PR/8/34), A/New Caledonia/20/99, A/Beijing/262/95, A/Johannesburg/282/96, A/Texas/36/91, A/California/969/09 A(H1N1)sov. Parmi les souches H3N2, on peut plus particulièrement citer : A/Panama/2007/99, A/Moscow/10/99, A/Johannesburg/33/94. Parmi les sous-types B du virus de l'influenza, on peut citer, à titre d'exemples, les sous-types B/Porto Rico/8/34, B/Johannesburg/5/99, B/Vienna/1/99, B/Ann Arbor/1/86, B/Memphis/1/93, B/Harbin/7/94, N/Shandong/7/97, B/Hong Kong/330/01, B/Yamanashi/166/98. Bien que tous les virus influenza, quelque soit leur origine soient visés par l'invention, l'invention trouve tout son intérêt pour les virus influenza humains et notamment circulant dans la population humaine. Selon un mode de réalisation particulier, le virus influenza est choisi parmi les virus A (H1N1) humains et les virus A (H3N2) humains. Dans le cadre de l'invention, le terme « virus » englobe les virus sauvages, les isolats viraux primaires obtenus à partir d'un individu infecté, les virus recombinants, les virus atténués, les virus réassortis, ainsi que les virus produits par génétique inverse. Les « semences vaccinales » qui sont utilisées pour la production de vaccin, sont, le plus souvent, obtenues par réassortiment génétique ou par génétique inverse et constituent donc un exemple particulier de virus au sens de l'invention.

Actuellement, le processus classique de production de vaccin repose dans un premier temps sur l'obtention par réassortiment génétique avec la souche A/PR8/34 (H1N1), de semences vaccinales sur oeuf, pour chacune des 3 souches prototypes annuelles déterminées par l'OMS pour chaque virus influenza. La plupart des fabricants de vaccin utilisent de manière usuelle ces virus réassortants dérivant principalement du virus parental A/PR/8/34. Ainsi, chaque semence vaccinale est issue d'un processus de réassortiment génétique entre la souche prototype et le virus A/PR8/34 (H1N1) qui possède des capacités réplicatives d'intérêt en oeuf.

Les particules virales des virus influenza sauvage (également nommés virus prototypes qui correspondent aux virus qui circulent dans la nature) contiennent huit segments de gène distincts constitués d'une chaîne unique d'ARN, chacun des gènes codant une à trois protéines déterminées du virus : HA, NA, M1, M2, NP, NS1, NEP, PB1, PB1F2, PB1N40, PB2 et PA. Les semences vaccinales obtenues par réassortiments correspondent à un réassortant vaccinal qui comporte au minimum les segments de gènes codant pour HA et NA de la souche prototype sur le fond génétique d'un virus prototype adapté pour la production à grande échelle, qui actuellement correspond à la souche parentale A/PR/8/34 (composition «6+2»). Les réassortants vaccinaux obtenus avec les 3 souches prototypes annuelles, nommés semences vaccinales, issus de ce réassortiment génétique peuvent ensuite être amplifiés sur oeuf ou en culture cellulaire. Aujourd'hui, le système oeuf est très largement utilisé à l'échelle industrielle. D'autres techniques pour la production de semences vaccinales, développées ces dernières années, reposent également sur la génétique inverse. La production des semences vaccinales par génétique inverse n'est, cependant, pas un procédé enregistré et agréé par l'OMS.

Quelle que soit la technique utilisée pour la production des semences vaccinales, les vaccins correspondent, le plus souvent, aux antigènes HA et NA qui sont purifiés à partir des productions de semences vaccinales en cellules allantoïques ou issues d'une culture cellulaire *in vitro.* Dans un vaccin, ces glycoprotéines peuvent être associées ou non à des adjuvants. Actuellement, les doses vaccinales sont définies par une quantité d'antigène HA fixe (15 microgrammes /sous-type viral/dose). Une détection positive de l'antigène NA est nécessaire pour la validation des lots vaccinaux.

Dans le cadre de l'invention, on entend par « antagoniste de Mdm2 », une molécule capable d'interagir avec Mdm2 et de bloquer, au moins pour partie, son activité biologique. En particulier, dans le cadre de l'invention, l'antagoniste de Mdm2 utilisé se comportera comme un inhibiteur de l'interaction entre la protéine Mdm2 et la protéine p53.

Un « inhibiteur de l'interaction entre la protéine Mdm2 et la protéine p53 » peut être défini comme un composé qui prévient l'interaction entre les protéines p53 et Mdm2. De tels composés, qui sont par exemple des desoxynucléotides antisens ciblant Mdm2, ou plus largement développés, des molécules de faibles poids moléculaires, peptidiques ou non peptidiques, se lient à l'interface entre p53 et Mdm2 et préviennent leur association.

La protéine suppressive de tumeur p53 joue un rôle central dans la régulation du cycle cellulaire, l'apoptose, la réparation de l'ADN et de nombreuses voies métaboliques. Par sa position centrale, p53 est la protéine la plus fréquemment mutée dans les tumeurs chez l'homme. En effet, près de 50% des cancers présentent des mutations du gène p53, résultant en l'inactivation ou la perte de la protéine. Plus largement, les fonctions de p53 sont la plupart du temps inhibées dans les cancers. En conditions normales, l'activité et la quantité de p53 sont étroitement régulées par la protéine Mdm2 (Murine double minute 2, ou appelée également Hdm2). Cette protéine ubiquitaire est une E3 ubiquitine ligase, entre autre capable d'interagir directement avec p53, la formation du complexe entre les deux protéines constituant une étape d'une boucle d'autorégulation : lorsque p53 est activée, elle se lie au promoteur P2 de Mdm2 et induit la transcription et l'expression de Mdm2. En retour, Mdm2 est, quant à elle, capable de se lier à p53 et de l'inhiber par différents mécanismes (inhibition activité transactivatrice de p53, délocalisation de p53 hors du noyau et dégradation de p53 via le protéasome) (Freedman et al. Cell Mol Life Sci. 1999 Jan. 55(1), 96-107).

Les propriétés les plus connues des antagonistes de Mdm2, inhibiteurs de l'interaction entre Mdm2 et p53 sont l'induction de l'activité de la protéine p53, mais aussi l'inhibition de la prolifération cellulaire. L'interaction entre les protéines p53 et Mdm2 implique les régions amino-terminales des deux partenaires. Ces régions d'interaction ont été déterminées par les méthodes de double hybride (levures) mais aussi par des expériences immunoprécipitation. Elles correspondent, sur p53, à la région 1 à 41/52 et sur Mdm2 à la région 1/19 à 118/202. Des expériences de mutagenèse dirigée ont montré plusieurs résidus sur p53 importants dans l'interaction avec Mdm2: Leu14, Phe19, Leu22 et Trp23 (Klein C, et al. Br. J. Cancer. 2004 Oct 18;91(8):1415-9).

La principale méthode pour évaluer l'efficacité de composés inhibiteur de l'interaction entre les protéines p53 et Mdm2 consiste à calculer une concentration inhibitrice à 50% (CI50 ou IC50 en anglais) et indirectement une constante d'inhibition (Ki, *via* l'équation de Cheng-Prusoff), en réalisant une courbe de réponse, en fonction du paramètre biologique que l'on cherche à inhiber, à savoir l'interaction p53/Mdm2.

Par exemple, il est possible de calculer les CI50 par mesure de résonance plasmonique de surface (SPR, de l'anglais « surface plasmon résonance », Biacore), en faisant rentrer en compétition la molécule inhibitrice avec Mdm2 : différentes concentration de composés sont incubées avec de la protéine Mdm2 recombinante et injectées dans une chambre (SPR) avec une puce sur laquelle est greffée de la protéine p53 recombinante, comme décrit par Vassilev et al, dans Science. 2004 Feb 6;303(5659):844-8. D'autres tests basés sur des techniques de mesures de fluorescence peuvent également être utilisés (Kane et al. Anal Biochem. 2000 Feb 1;278(1):29-38, Lu et al. J Med Chem. 2006 Jun 29;49(13):3759-62). Dans le cas de la famille de molécules telle que la Nutlin-3, les composés ont une CI50 d'environ 100-300nm (Vassilev *et al.* 2004, *supra*). Dans le cas de la molécule NSC66811, la constante Ki est de 120nM (Lu *et al.* 2006, *supra*).

Dans de telles expériences de compétition, il est possible d'introduire comme contrôle un peptide naturel dérivé de p53, et notamment le peptide correspondant aux résidus 13 à 29 de p53 et de **SEQ ID N°1 :** PLSQETFSDLWKLLPEN-NH2 tel que décrit par Lu *et al.* 2006, *supra* dont la constante Ki mesurée et donnée dans cette publication est de 6670nM. Sont notamment considérés comme des inhibiteurs de l'interaction Mdm2/p53 dans le cadre de l'invention, les composés qui présentent un Ki ou une IC50 inférieur à ce peptide, sur au moins un des tests de compétition décrits dans Vassilev *et al.* 2004, Kane *et al.* 2000, Lu *et al.* 2006 *supra.* De manière plus générale, un composé présentant, sur le test de compétition décrit dans Lu *et al.* 2006 *supra* et dans le supporting information JM060023, un Ki inférieur à 10 µM, de préférence inférieur à 5 µM, et encore plus préférentiellement inférieur à 1 µM, seront préférés.

Une revue des inhibiteurs utilisés dans le cadre de l'invention est donnée dans les publications de Lutz Weber dans Expert Opin. Ther. Patents 2010, 20(2), 179-190 et de Shangary et al. dans Annu Rev Pharmacol Toxicol. 2009;49:223-41, auxquelles on pourra se référer pour plus de détails.

Dans le cadre de l'invention, on utilisera :
- la Nutlin-3, (±)-4-[4,5-bis(4-chlorophényl)-2-(2-isopropoxy-4-méthoxy-phényl)-4,5-dihydro-imidazole-1-carbonyl]-pipérazin-2-one, décrite par Vassilev et al. Science. 2004 Feb 6, 303(5659), 844-8 de formule : 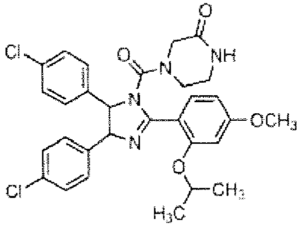 qui fait l'objet de deux essais cliniques de phase I, en oncologie,
- la molécule NSC66811 (2-méthyl-7-[phényl(phénylamino)méthyl]-8-quinolinol) de formule : 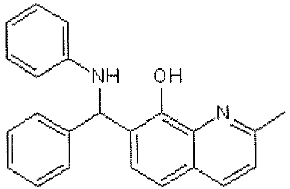
- et les molécules de formule suivante :

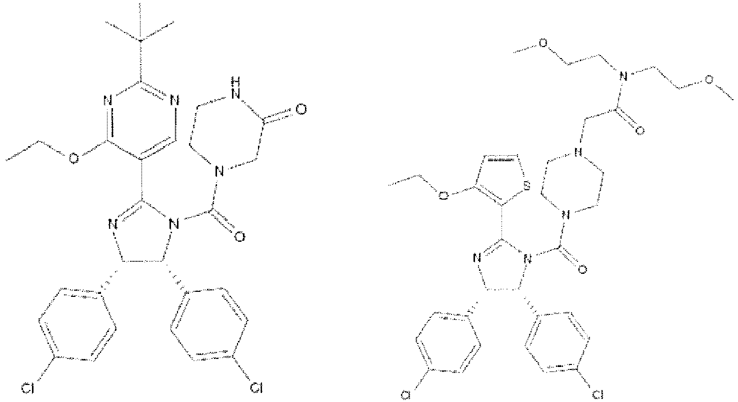

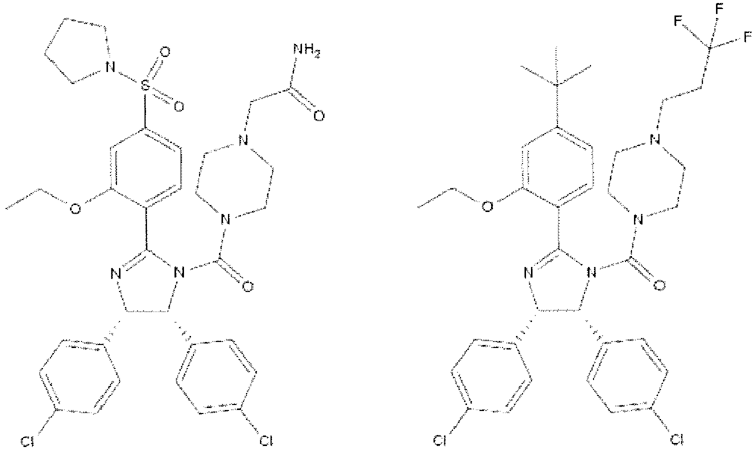

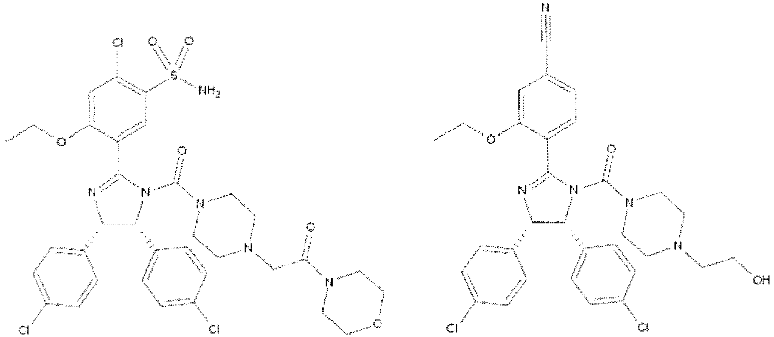

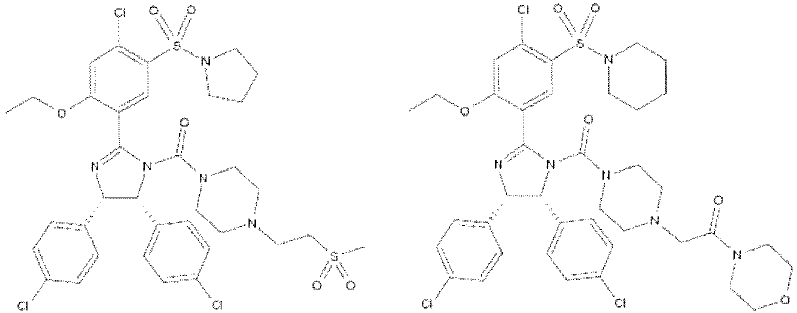

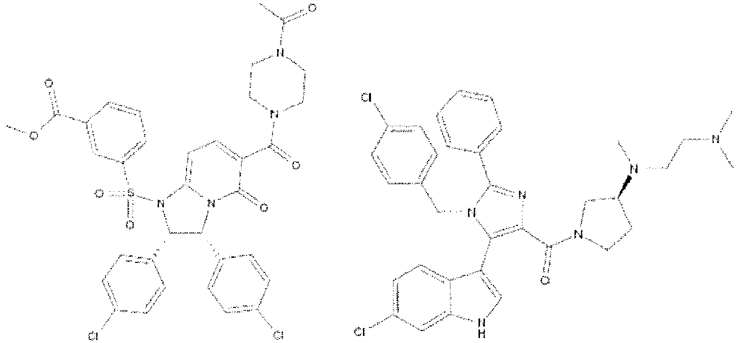

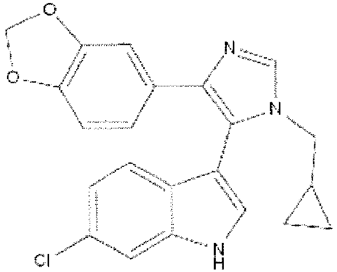

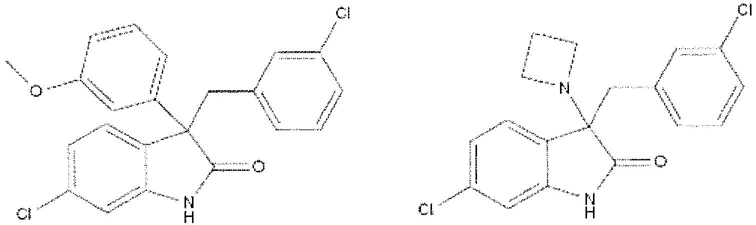

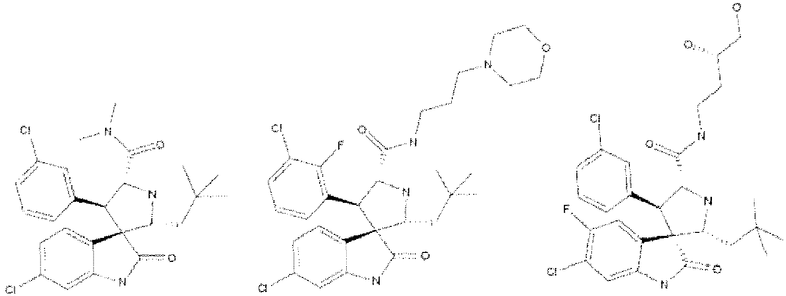

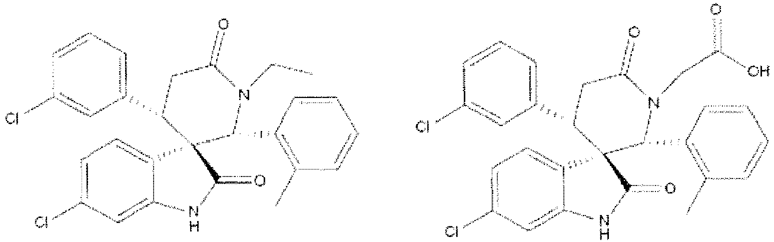

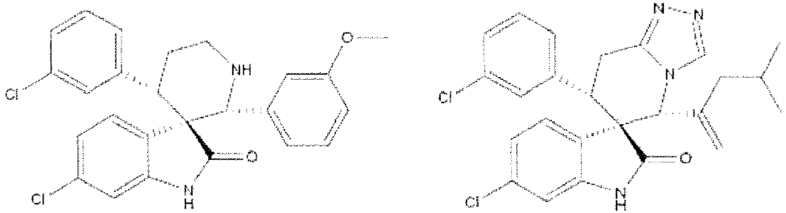

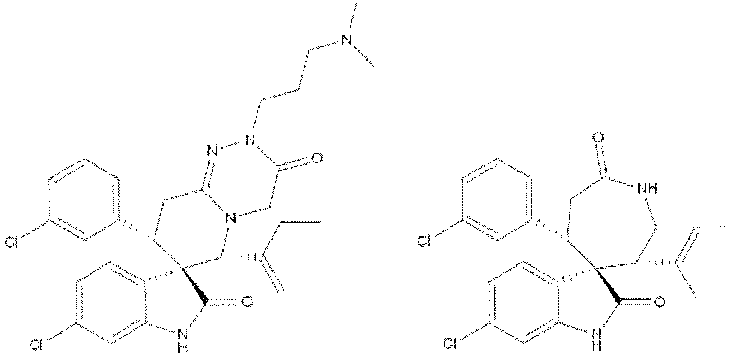

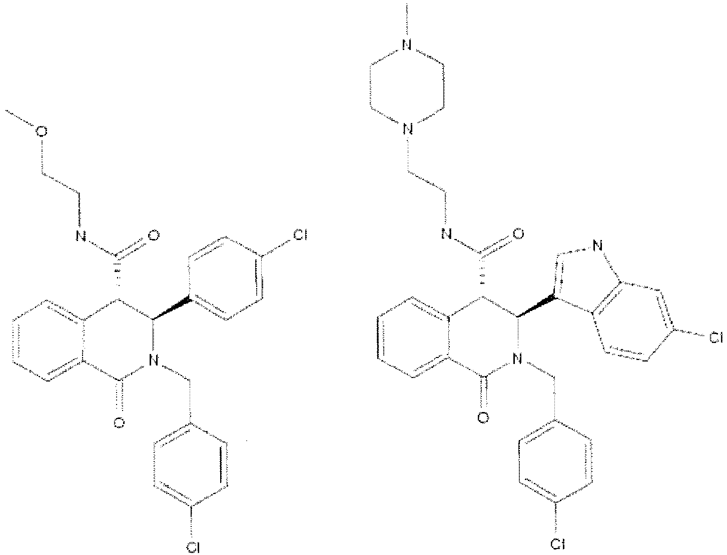

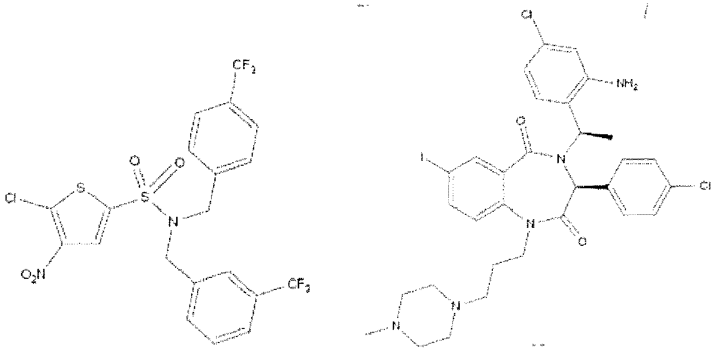

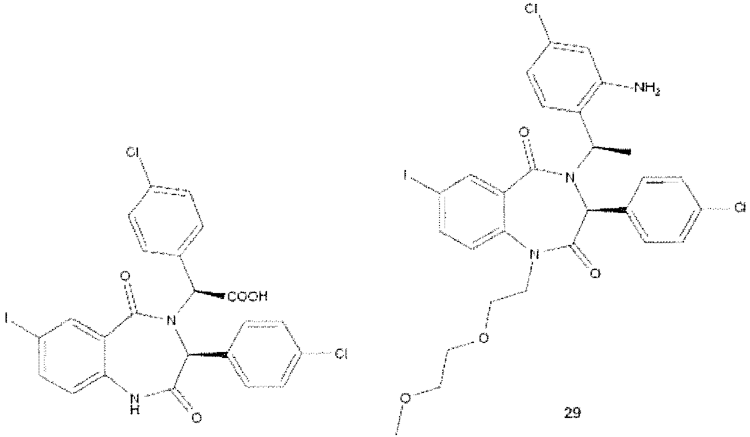

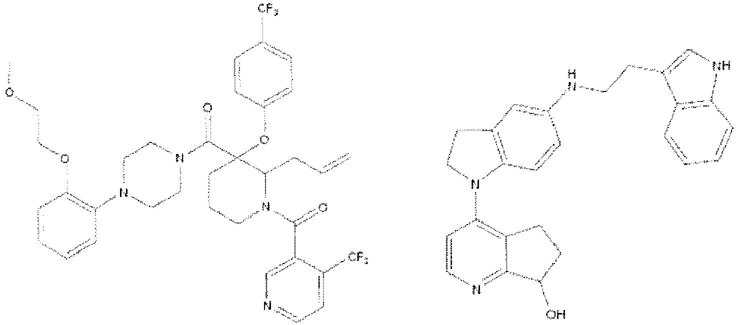

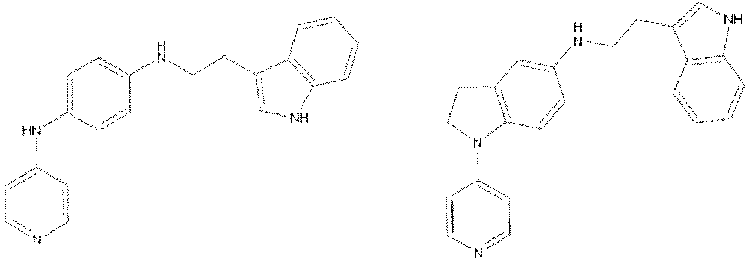

De telles molécules sont connues pour leur application dans le traitement du cancer, mais leur utilisation pour améliorer quantitativement la production de virus influenza n'a jamais été envisagée. L'apport de l'invention réside dans l'utilisation telle que définie à la revendication 1, d'un antagoniste de Mdm2, inhibiteur de l'interaction entre la protéine Mdm2 et la protéine p53, pour la production de virus ou de semences vaccinales influenza. Toutes techniques connues pour leur production peuvent être utilisées.

La production des virus ou semences vaccinales influenza est réalisée *in vitro* en culture cellulaire, dans une lignée cellulaire MDCK, en présence d'un antagoniste de Mdm2, inhibiteur de l'interaction entre la protéine Mdm2 et la protéine p53 tel que défini à la revendication 1.

Plusieurs paramètres qui seront ajustés par l'homme du métier peuvent avoir une influence sur l'effet de l'antagoniste de Mdm2, inhibiteur de l'interaction entre la protéine Mdm2 et la protéine p53 : quantité d'inhibiteur, moment de l'adjonction de l'inhibiteur, quantité initiale de virus au moment de l'infection, temps de production.

Par exemple, l'antagoniste de Mdm2, inhibiteur de l'interaction entre la protéine Mdm2 et la protéine p53, sera utilisé à une concentration de 0,1 à 100 µM. Notamment, lorsqu'un dérivé d'imidazole, telle que la Nutlin-3, sera utilisé, une concentration de l'ordre de 1 µM à 10 µM sera, de préférence, choisie, et lorsqu'un dérivé de quinoline, tel que le NSC66811, sera utilisé, une concentration de l'ordre de 0,5 µM à 5 µM sera, de préférence, choisie.

Le système cellulaire sélectionné correspondant à culture cellulaire de MDCK est infecté avec un virus ou une semence vaccinale influenza. L'adjonction de l'antagoniste de Mdm2, inhibiteur de l'interaction entre la protéine Mdm2 et la protéine p53 peut se faire, avant, après ou en même temps que l'infection. Les cellules MDCK de chien sont appropriées pour la culture cellulaire, la réplication, la production de virus et la production d'antigènes viraux.

Les cellules MDCK sont par exemple décrites dans US2006/0188977 et EP1862537. On citera en particulier les lignées suivantes : MDCK-SF101 (ATCC PTA-6501), MDCK-SF102 (ATCC PTA-6502), MDCK-SF103 (ATCC PTA-6503), les cellules MDCK de la lignée CCL-34 *Canis familiaris* MDCK (NBL-2).

Avantageusement, les cellules sont choisies parmi les lignées cellulaires bénéficiant d'autorisations réglementaires pour la production virale en culture cellulaire à des fins médicales.

Selon un mode de réalisation particulier, le procédé de production de virus influenza selon l'invention comprend les étapes suivantes : a) l'infection avec un virus influenza, et en particulier avec des semences vaccinales influenza, d'un système cellulaire sélectionné qui est des cellules de la lignée cellulaire MDCK dans un milieu de culture cellulaire; b) l'incubation du système cellulaire de production sélectionné et infecté à l'étape a) dans des conditions permettant la réplication dudit virus influenza, et en particulier des semences vaccinales influenza; c) la récolte du virus influenza entier, et en particulier des semences vaccinales, produits, notamment, dans le surnageant de culture et/ou dans lesdites cellules en culture.

L'utilisation d'un antagoniste de Mdm2, inhibiteur de l'interaction entre la protéine Mdm2 et la protéine p53 de la revendication 1 peut être mise en oeuvre dans la production de toute sorte de virus influenza, notamment d'un virus sauvage, d'un isolat viral primaire obtenu à partir d'un individu infecté, d'un virus recombinant, d'un virus atténué, d'un virus réassorti, ou d'un virus produit par génétique inverse.

La production est réalisée dans le système cellulaire sélectionné, *in vitro.* L'infection des cellules d'une lignée cellulaire MDCK avec le virus influenza sélectionné est réalisée dans un milieu de culture cellulaire qui est, de préférence, un milieu sans sérum, et de manière encore plus préféré, dépourvu de protéines d'origine animale (milieu synthétique calibré). Selon un mode particulier de mise en oeuvre, la culture des cellules infectées est réalisée en présence d'une enzyme protéolytique dans le milieu de culture, dans des conditions suffisantes pour assurer la propagation du virus dans la culture. La dite enzyme protéolytique est, par exemple, sélectionnée parmi la trypsine, la chymotrypsine, la thermolysine, la pepsine, la pancréatine, la papaïne, la pronase, la subtilisine A, l'élastase, la furine et la carboxypeptidase. De façon avantageuse, cette enzyme est une enzyme recombinante et n'est donc pas d'origine animale.

Le procédé selon l'invention peut être appliqué à la production de virus influenza réassortants, du fait du caractère fragmenté des virus influenza. Dans ce cas, le système cellulaire de cellules MDCK sélectionné est infecté, avec au moins deux souches de virus influenza, ce qui conduit à la production d'un mélange de segments dérivés des deux souches de virus dans une seule cellule hôte. Durant l'assemblage des virus, toutes les combinaisons de fragments sont théoriquement possibles. Les nouvelles combinaisons sont appelées réassortants. Des réassortants particuliers peuvent être sélectionnés par suppression ou élimination des autres virus, par exemple au moyen d'anticorps. C'est selon ce type de procédé que les semences vaccinales sont générées. Pour plus de détails sur ces techniques, on pourra se référer à Kilnourne E.D. in Plotkin SA and Mortimer E.A. Eds, Vaccines 1994.

Le procédé selon l'invention peut également être appliqué à la production de virus influenza par génétique inverse. En particulier, des plasmides d'expression peuvent être transfectés dans les cellules et permettent ainsi la production de virus recombinant (J Gen Virol. 2000 Dec;81(Pt 12):2843-7). Une autre technique moins répandue consiste à utiliser des transcrits modifiés des ARN viraux du virus influenza qui sont transcrits *in vitro* à partir de construction d'ADNc en présence de protéines, notamment, NP, PB1, PB2 et PA purifiées. Les ribonucléoparticules synthétiques ainsi produites sont ensuite transfectées dans des cellules préalablement infectées avec un virus influenza. Pour plus de détails sur cette dernière technique, on pourra se référer à Enami, Proc. Natl. Acad. Sci. 8è, 1990, 3802-3805, à Enami et Palese, J. Virol. 1991, 65, 2511-2513 (1991) et à Luytjes, Cell. 1989, 59, 1107-1113.

Dans le cadre de l'invention, selon un mode de mise en oeuvre particulier, le procédé de préparation d'un virus influenza, et en particulier d'une semence vaccinale selon l'invention peut comprendre une étape d'inactivation du virus récolté. L'inactivation peut être réalisée selon toute technique connue, et, par exemple, grâce à un traitement avec du formaldéhyde, de la béta-propiolactone, de l'éther, de l'éther avec un détergent (tel que le Tween-80), du bromure de cétyl-triméthylammonium (CTAB) du Triton N102, du deoxycholate de sodium ou du tri(Nbutyl)phosphate.

Le procédé de production de virus et en particulier de semence vaccinale selon l'invention peut être inclus dans un procédé de fabrication de vaccin. Dans ce cas, le procédé de préparation d'un vaccin selon l'invention comprend une étape de production de protéines antigéniques de surface (HA et NA) à partir d'un virus entier d'influenza produit. Dans ce cas, il peut être nécessaire de traiter le milieu, notamment le surnageant, contenant le virus entier obtenu, avec un enzyme de digestion des acides désoxyribonucléiques (ADN), par exemple des enzymes DNases ou nucléases. Il est également possible d'ajouter un détergent cationique, tel qu'un sel de cétyl-triméthyl ammonium ou de myristyl-triméthyl ammonium, la lipofectine ou la lipofectamine.

La récolte du virus ou des protéines de surface, s'accompagne le plus souvent d'une concentration et/ou purification et est réalisée selon des techniques bien connues de l'homme de l'art, mettant en oeuvre notamment l'ultrafiltration ou la centrifugation (comme décrit, par exemple, dans Furminger, in Nicholson, Webster and Hay (Eds.), Textbook of influenza, chapitre 24 pp324-332).

Le vaccin obtenu grâce au procédé selon l'invention peut correspondre à des pathogènes tués (également nommés inactivés) ou à des pathogènes vivants atténués. Le vaccin peut, par exemple, correspondre au surnageant de culture obtenu après la production du virus ou encore aux protéines antigéniques de surface susceptibles d'être obtenues à partir du virus produit conformément au procédé selon l'invention.

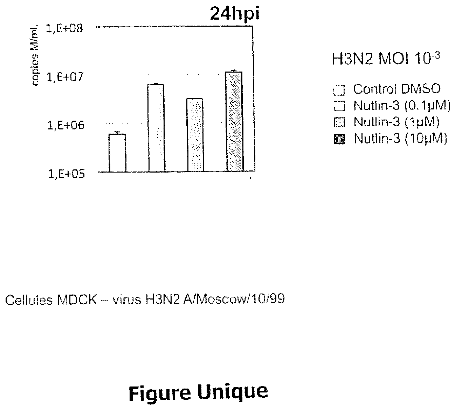

L'exemple ci-après, en référence à la Figure unique annexée, permet d'illustrer l'invention, mais n'a aucun caractère limitatif.

La **Figure unique** présente les quantités observées de copies de génome viral M/mL dans le surnageant de culture (M pour le segment de génome viral codant les protéines virales M1 et M2), avec différentes concentrations en nutlin-3.

Les expériences ont été réalisées à une température de 37°C. Le milieu de culture utilisé est le DMEM (Ref 41966 GIBCO), supplémenté en trypsine (1ug/mL SIGMA) lors des infections.

### Effet de la Nutlin-3 sur les titres de virus produits - Evaluation de l'effet des différentes concentrations de Nutlin-3

### Test réalisé sur des cellules MDCK avec le virus H3N2 A/Moscow/10/99

La quantité de copies M/mL de surnageant a été mesurée par RT-qPCR après plusieurs cycles viraux (24hpi). Les cellules ont été mises en présence de Nutlin-3, 14h avant l'infection (T = - 14h),. Dans tous les cas, l'addition de Nutlin-3 se fait en une seule fois.

La lignée MDCK (ATCC n° CCL-34) est la lignée la plus largement utilisée pour la production de virus influenza en laboratoire. C'est une lignée cellulaire qui est également enregistrée et avec laquelle des lots de vaccin ont été produits par Novartis et Solvay.

Les résultats présentés sur la **Figure unique** indiquent qu'à une MOI initiale de 10⁻³, la molécule Nutlin-3 à différentes concentration (0,1 ; 1 et 10 µM), avec un prétraitement 14h avant l'infection, augmente de manière forte le titre viral (copies génome M/mL) dans les surnageants cellulaires après plusieurs cycles viraux consécutifs. A 24hpi, on obtient jusqu'à 19 fois plus de copies de génome M/mL en présence de Nutlin-3 par rapport au contrôle.

Ces expériences montrent un net effet de la Nutlin-3 sur la production virale des virus influenza, sur MDCK.

## Revendications

1. Procédé de production d'un virus influenza, et notamment de semences vaccinales, dans lequel la production est réalisée *in vitro* en culture cellulaire, dans une lignée cellulaire MDCK, **caractérisé en ce que** la production est réalisée en présence d'un antagoniste de Mdm2, inhibiteur de l'interaction entre la protéine Mdm2 et la protéine p53, choisi parmi :
- la Nutlin-3, (±)-4-[4,5-bis(4-chlorophényl)-2-(2-isopropoxy-4-méthoxy-phényl)-4,5-dihydro-imidazole-1-carbonyl]-pipérazin-2-one de formulé : 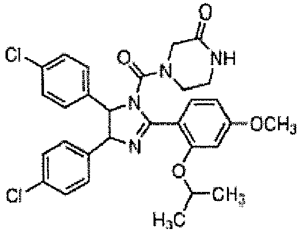
- la molécule NSC66811 (2-méthyl-7-[phényl(phénylamino)méthyl]-8-quinolinol) de formule : 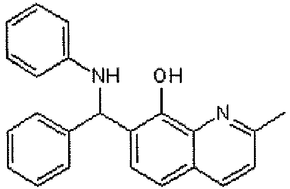
- et les molécules de formule suivante :

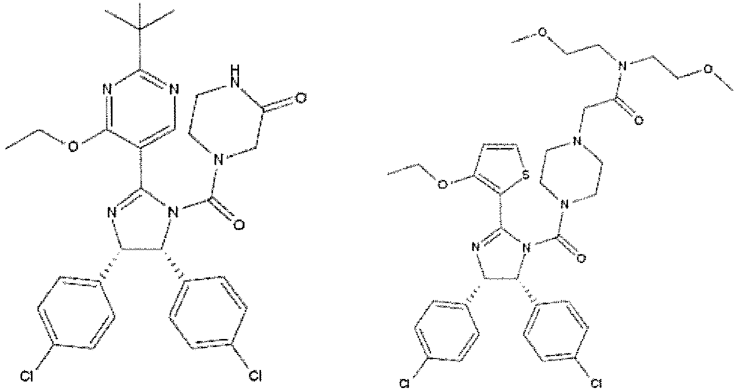

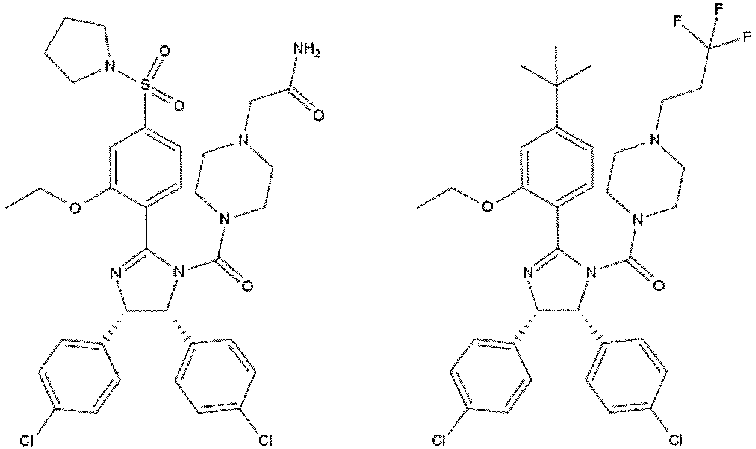

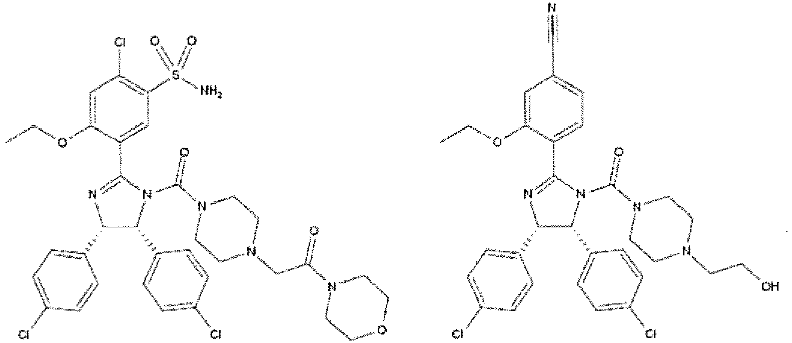

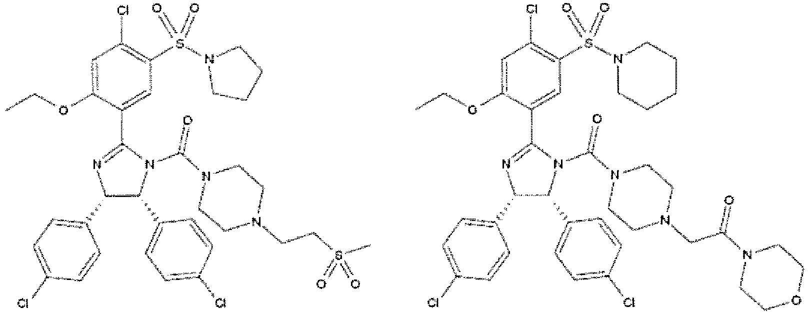

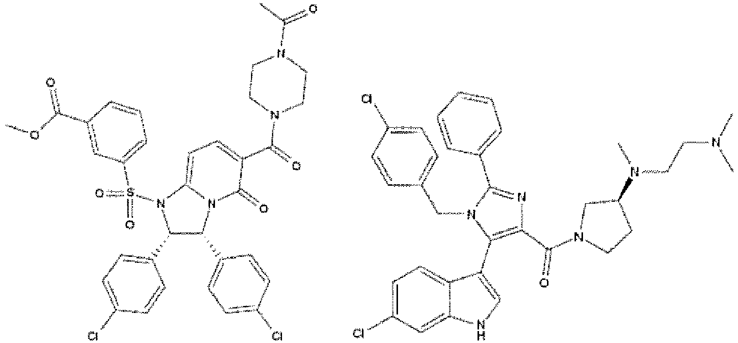

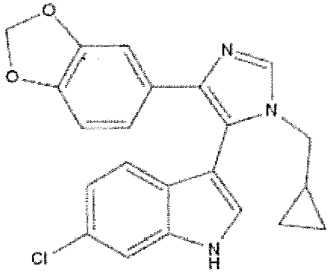

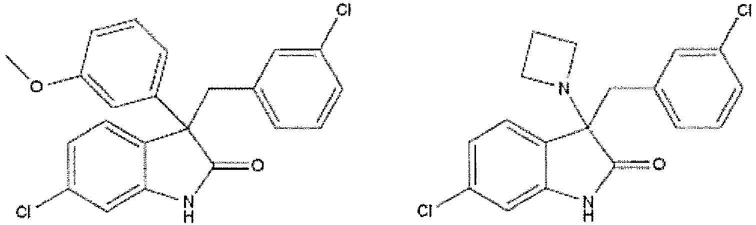

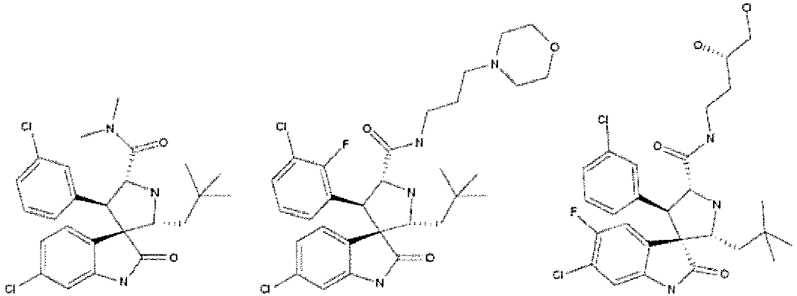

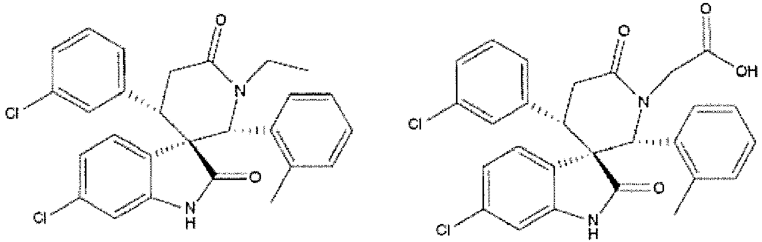

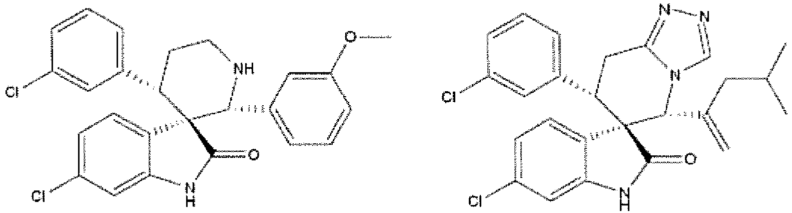

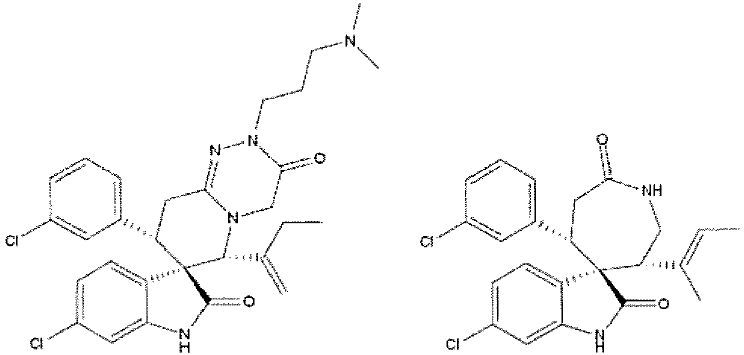

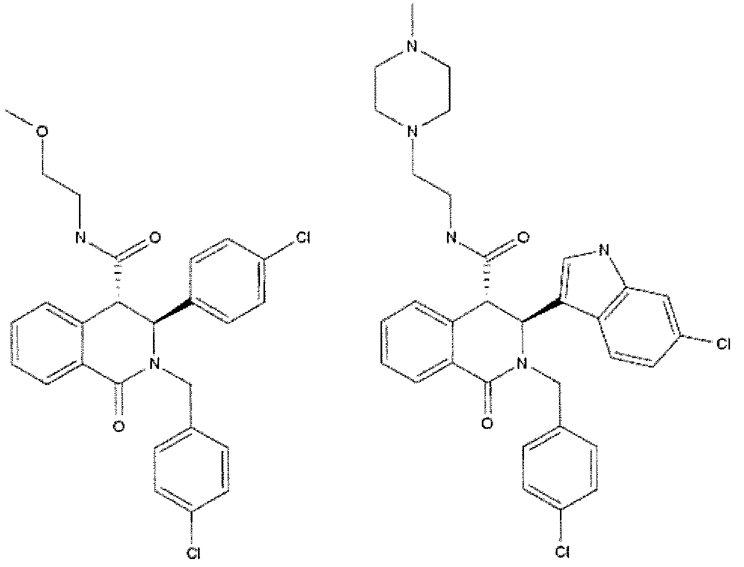

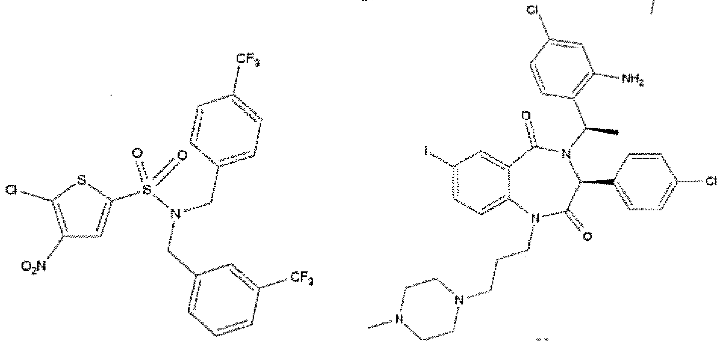

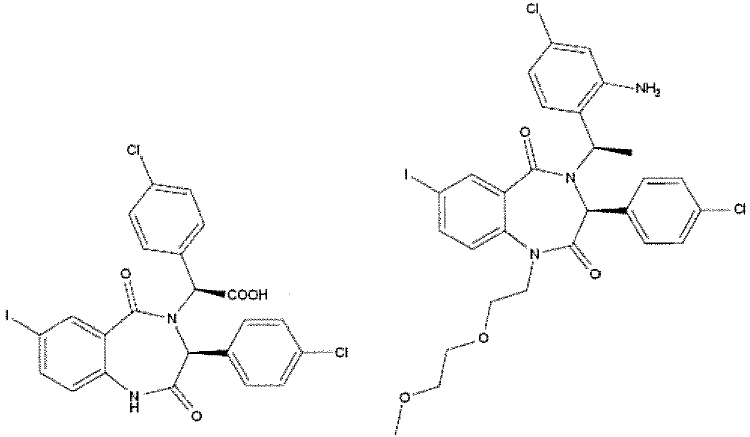

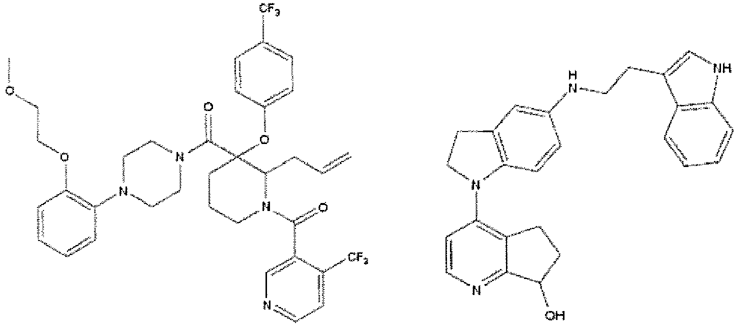

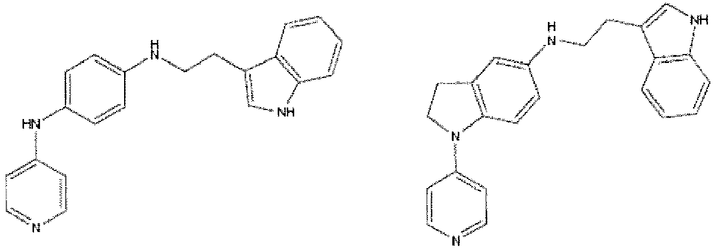

2. Procédé de production d'un virus influenza selon la revendication 1 **caractérisé en ce que** l'antagoniste de Mdm2 est utilisé pour améliorer quantitativement la production du virus influenza produit.

3. Procédé de production d'un virus influenza selon la revendication 1 ou 2 **caractérisé en ce que** le virus influenza est choisi parmi les sous-types A, B et C, et notamment les virus influenza de sous-type A correspondant aux souches H1N1, H2N2, H3N2, H4N2, H4N6, H5N1, H5N2, H7N7 et H9N2, et en particulier le virus A H3N2.

4. Procédé de production d'un virus influenza selon l'une des revendications 1 à 3 **caractérisé en ce que** la production est réalisée dans la lignée cellulaire MDCK qui est infectée avec un virus influenza et **en ce que** l'antagoniste de Mdm2 est ajouté, avant, après ou en même temps que l'infection.

5. Procédé de production d'un virus influenza selon l'une des revendications précédentes comprenant les étapes suivantes : a) l'infection avec un virus infiluenza, et en particulier avec des semences vaccinales influenza, des cellules de la lignée cellulaire MDCK dans un milieu de culture cellulaire; b) l'incubation de la lignée cellulaire MDCK de production sélectionnée et infectée à l'étape a) dans des conditions permettant la réplication dudit virus influenza, et en particulier des semences vaccinales influenza; c) la récolte du virus influenza entier, et en particulier des semences vaccinales, produits dans le surnageant de culture et/ou dans lesdites cellules en culture.

6. Procédé de production d'un virus influenza selon la revendication 5 **caractérisé en ce qu'**il comprend l'inactivation du virus récolté à l'étape c).

7. Procédé de production d'un virus influenza selon l'une des revendications précédentes **caractérisé en ce que** le virus est un virus sauvage, un isolat viral primaire obtenu à partir d'un individu infecté, un virus recombinant, un virus atténué, un virus réassorti, ou un virus produit par génétique inverse.

8. Procédé de préparation d'un vaccin contre un virus influenza **caractérisé en ce qu'**il comprend la production d'un virus influenza par un procédé selon l'une des revendications 1 à 7.

9. Procédé de préparation d'un vaccin selon la revendication 8 **caractérisé en ce qu'**il comprend la production de protéines antigéniques de surface à partir d'un virus entier d'influenza produit selon le procédé de l'une des revendications 1 à 7.

10. Procédé selon la revendication 8 ou 9 **caractérisé en ce que** le vaccin obtenu correspond à des pathogènes tués ou à des pathogènes vivants atténués.

## Patentansprüche

1. Verfahren zur Herstellung eines Influenzavirus und insbesondere von Impfkristallen, bei dem die Herstellung *in vitro* in Zellkultur, in einer MDCK Zelllinie vollzogen wird, **dadurch gekennzeichnet, dass** die Herstellung in Gegenwart eines Mdm2 Antagonisten, Inhibitor der Interaktion zwischen dem Mdm2 Protein und dem p53 Protein, vollzogen wird, der ausgewählt ist aus:
- Nutlin-3, (±)-4-[4,5-bis(4-Chlorophenyl)-2-(2-isopropoxy-4-methoxy-phenyl)-4,5-dihydro-imidazol-1-carbonyl]-piperazin-2-on der Formel: 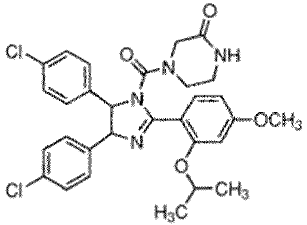
- dem Molekül NSC66811 (2-Methyl-7-[phenyl(phenylamino)methyl]-8-chinolinol) der Formel: 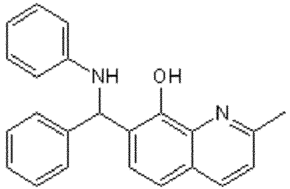
- und den Molekülen der folgenden Formel:

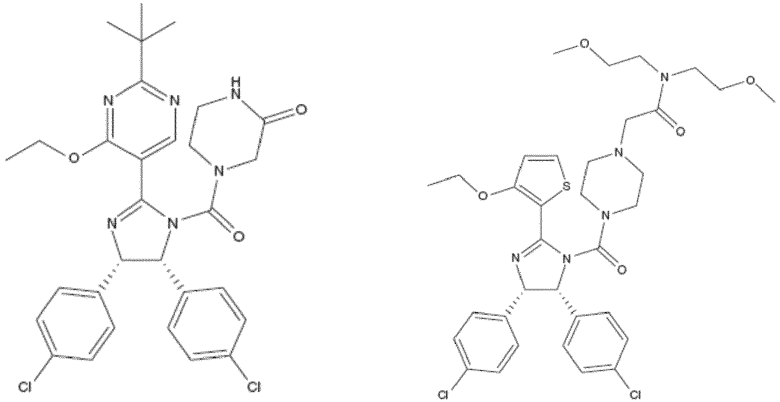

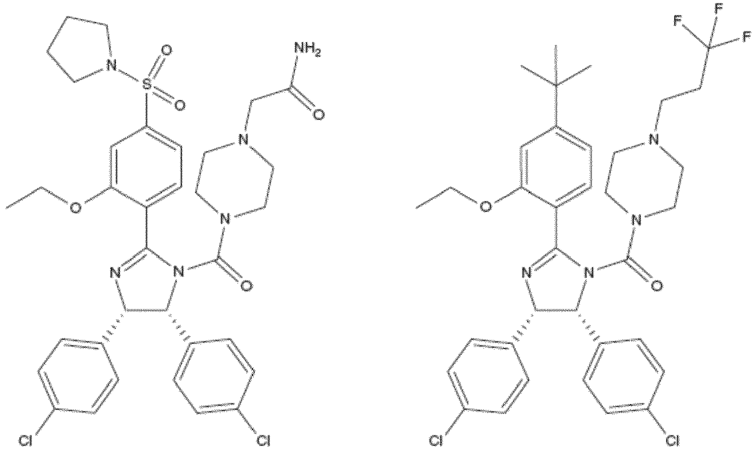

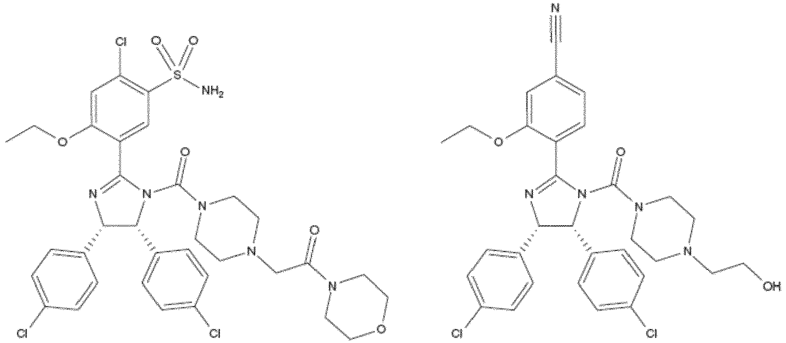

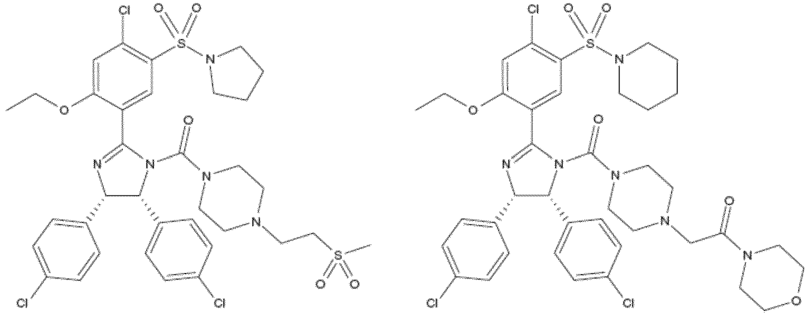

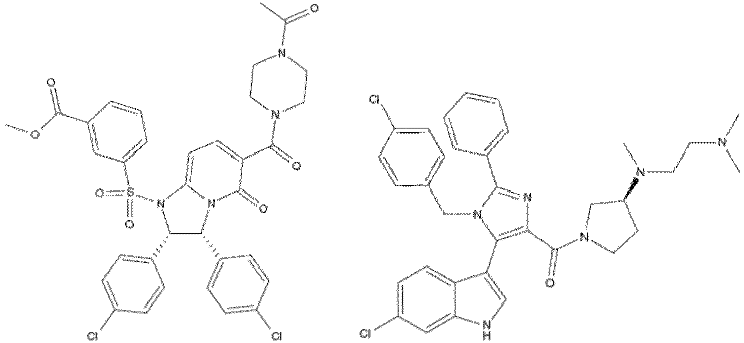

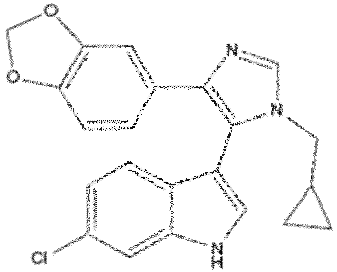

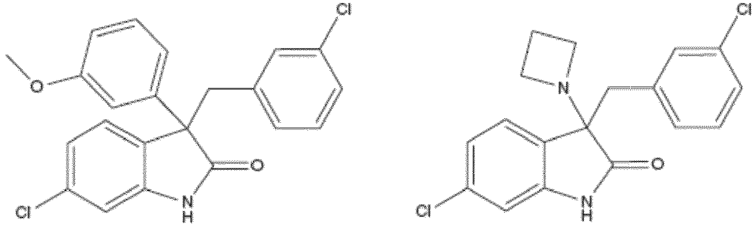

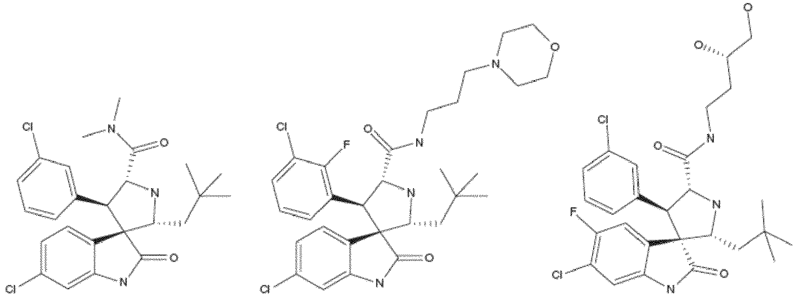

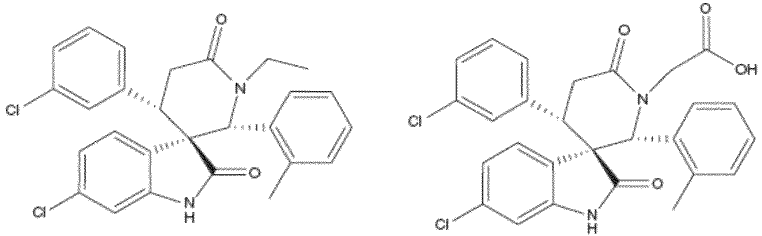

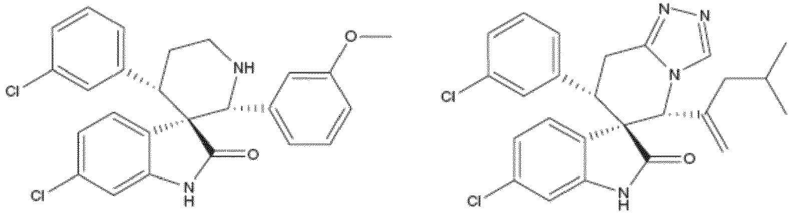

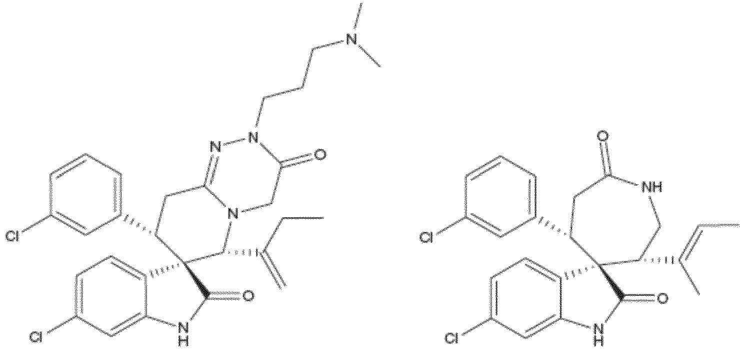

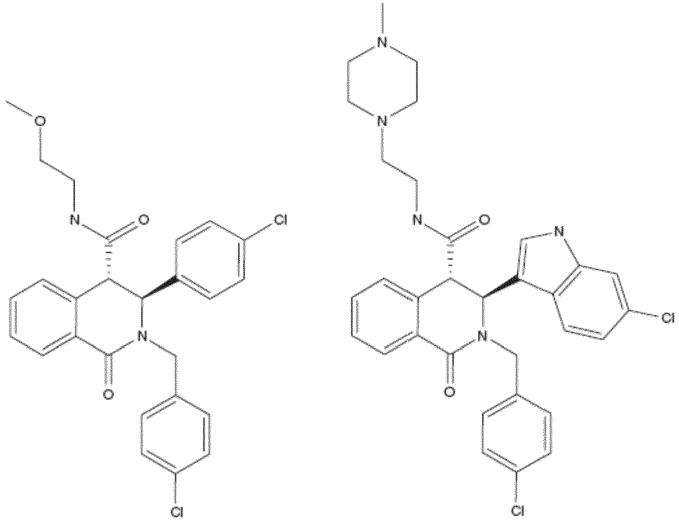

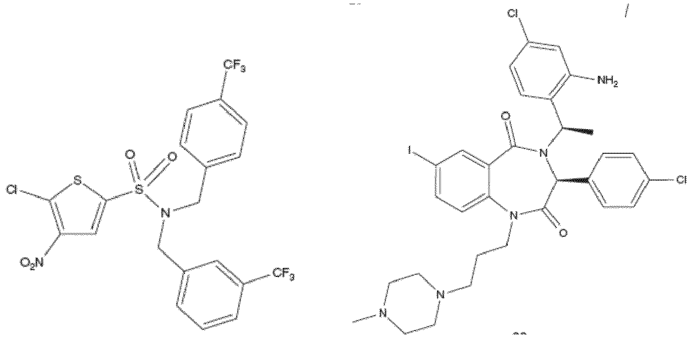

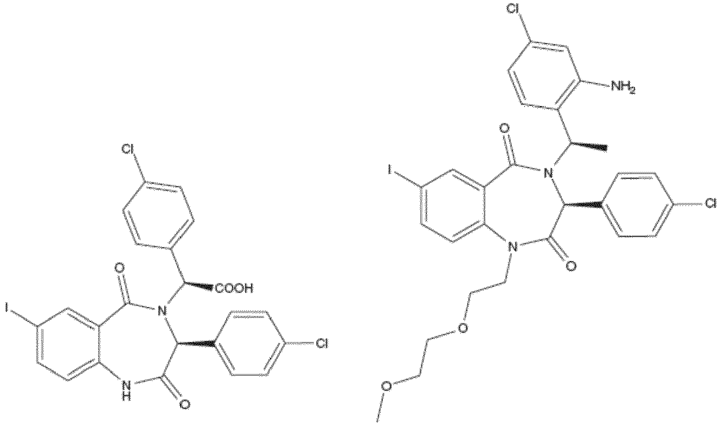

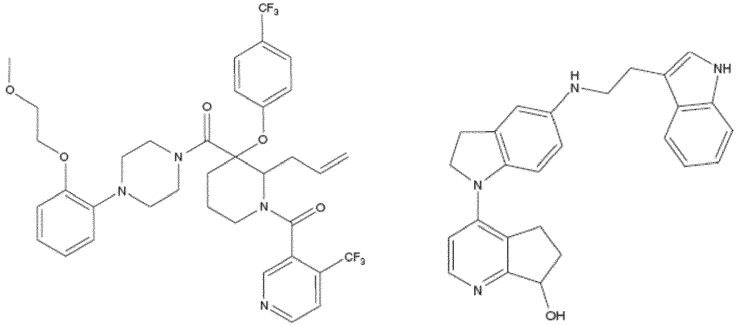

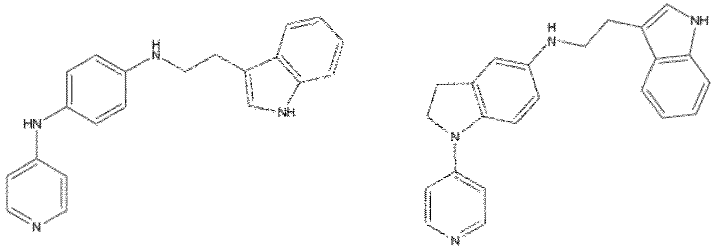

2. Verfahren zur Herstellung eines Influenzavirus nach Anspruch 1, **dadurch gekennzeichnet, dass** der Mdm2 Antagonist verwendet wird, um die Herstellung des erzeugten Influenzavirus quantitativ zu verbessern.

3. Verfahren zur Herstellung eines Influenzavirus nach Anspruch 1 oder 2, **dadurch gekennzeichnet, dass** das Influenzavirus aus den Subtypen A, B und C ausgewählt ist, und insbesondere den Influenzaviren des Subtyps A, die den Stämmen H1 N1, H2N2, H3N2, H4N2, H4N6, H5N1, H5N2, H7N7 und H9N2 entsprechen, und insbesondere dem Virus A H3N2.

4. Verfahren zur Herstellung eines Influenzavirus nach einem der Ansprüche 1 bis 3, **dadurch gekennzeichnet, dass** die Herstellung in der MDCK Zelllinie, die mit einem Influenzavirus infiziert ist, vollzogen wird und dass der Mdm2 Antagonist vor, nach oder gleichzeitig mit der Infektion zugegeben wird.

5. Verfahren zur Herstellung eines Influenzavirus nach einem der vorhergehenden Ansprüche, das die folgenden Schritte umfasst: a) das Infizieren der Zellen der MDCK Zelllinie, in einem Zellkulturmedium, mit einem Influenzavirus und insbesondere mit Influenza-Impfkristallen, b) das Inkubieren der ausgewählten und bei Schritt a) infizierten Herstellungs-MDCK Zelllinie unter Bedingungen, die die Replikation des Influenzavirus und insbesondere der Influenza-Impfkristalle ermöglichen, c) das Ernten des vollständigen Influenzavirus und insbesondere der Impfkristalle, die im Kulturüberstand und/oder in den kultivierten Zellen erzeugt werden.

6. Verfahren zur Herstellung eines Influenzavirus nach Anspruch 5, **dadurch gekennzeichnet, dass** es die Inaktivierung des bei Schritt c) geernteten Virus umfasst.

7. Verfahren zur Herstellung eines Influenzavirus nach einem der vorhergehenden Ansprüche, **dadurch gekennzeichnet, dass** das Virus ein Wildstamm-Virus, ein aus einem infizierten Individuum gewonnenes primäres Virus-Isolat, ein rekombinantes Virus, ein abgeschwächtes Virus, ein reassortiertes Virus oder ein durch reverse Genetik erzeugtes Virus ist.

8. Verfahren zur Herstellung eines Impfstoffes gegen ein Influenzavirus, **dadurch gekennzeichnet, dass** es die Herstellung eines Influenzavirus durch ein Verfahren nach einem der Ansprüche 1 bis 7 umfasst.

9. Verfahren zur Herstellung eines Impfstoffes nach Anspruch 8, **dadurch gekennzeichnet, dass** es die Herstellung von antigenen Oberflächenproteinen aus einem nach dem Verfahren von einem der Ansprüche 1 bis 7 erzeugten vollständigen Influenzavirus umfasst.

10. Verfahren nach Anspruch 8 oder 9, **dadurch gekennzeichnet, dass** der gewonnene Impfstoff toten Pathogenen oder abgeschwächten lebenden Pathogenen entspricht.

## Claims

1. A method for producing an influenza virus, and in particular vaccine seeds, in which the production is carried out *in vitro* in cell culture, in a MDCK cell line, **characterized in that** the production is carried out in the presence of an Mdm2 antagonist, inhibitor of the interaction between the Mdm2 protein and the p53 protein, chosen from:
- Nutlin-3, (±)-4-[4,5-bis(4-chlorophenyl)-2-(2-isopropoxy-4-methoxy-phenyl)-4,5-dihydroimidazole-1-carbonyl]piperazin-2-one of formula: 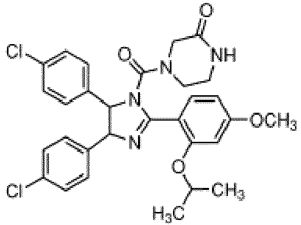
- the NSC66811 molecule (2-methyl-7-[phenyl(phenylamino)methyl]-8-quinolinol) of formula: 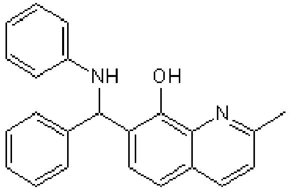
- and the molecules having the following formula:

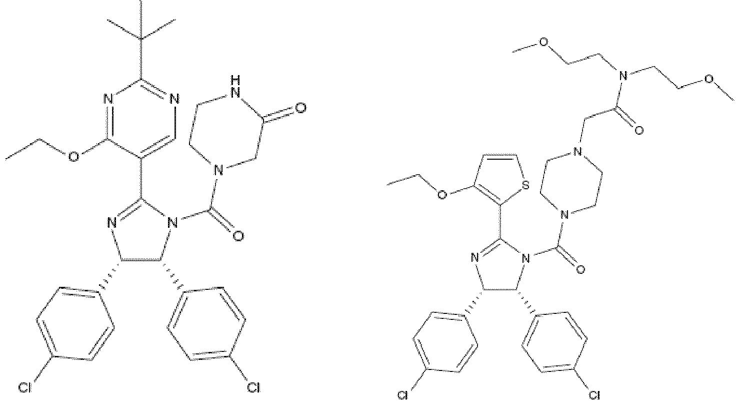

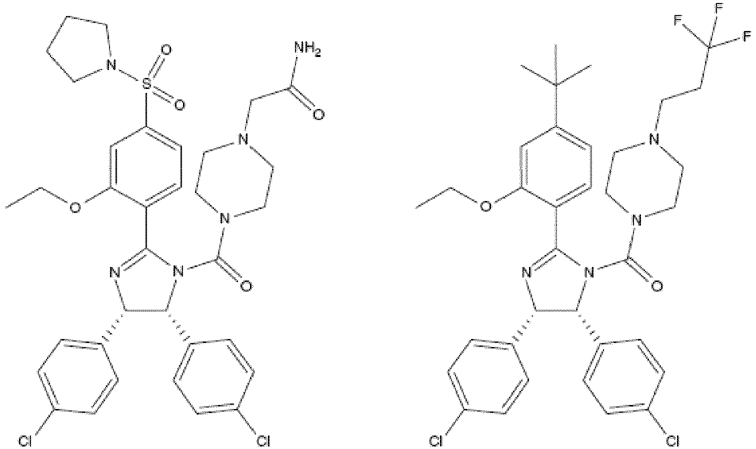

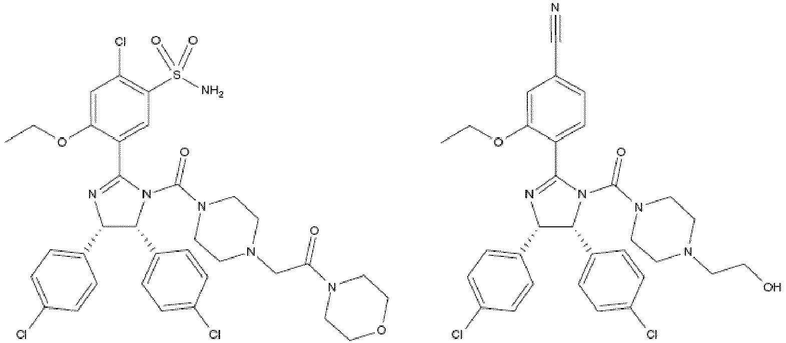

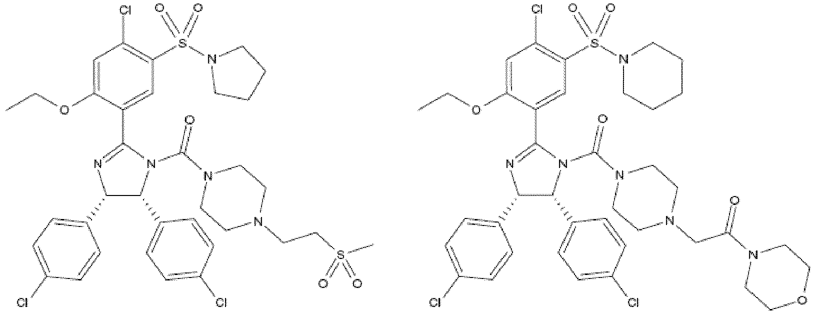

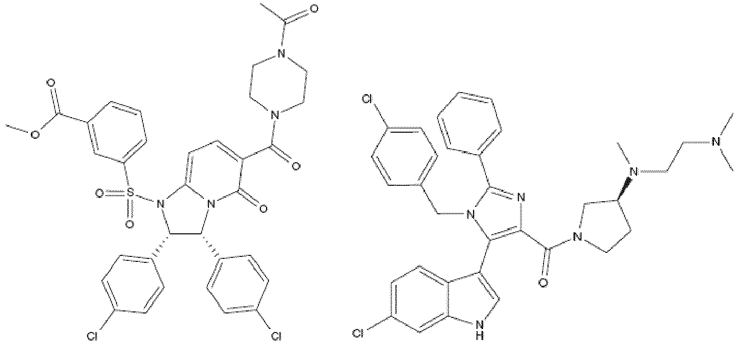

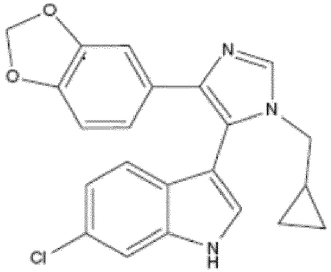

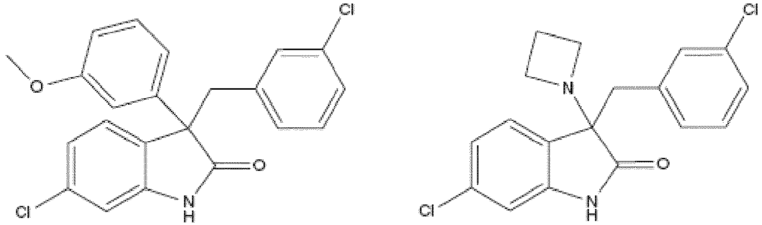

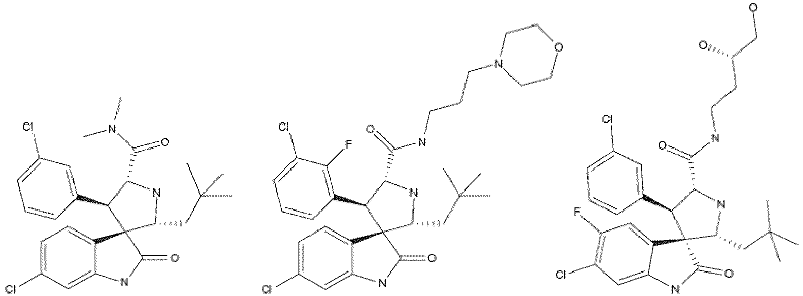

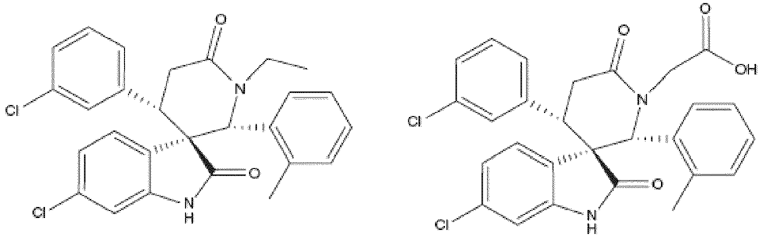

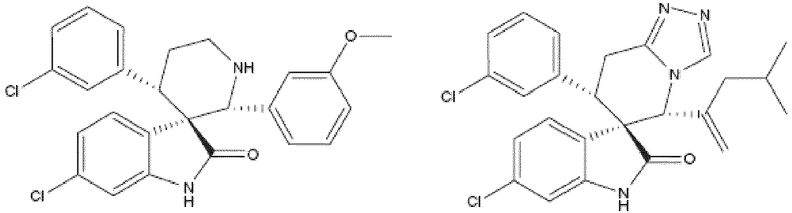

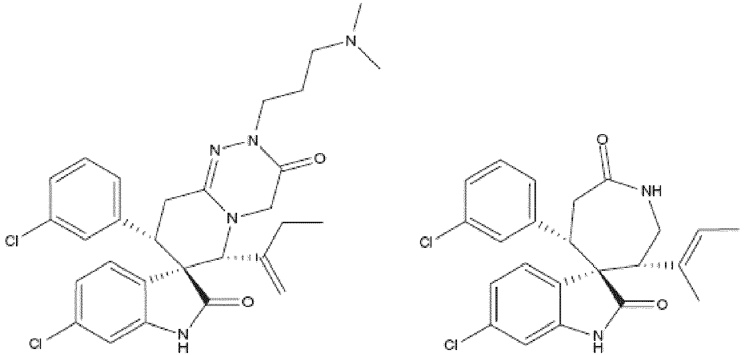

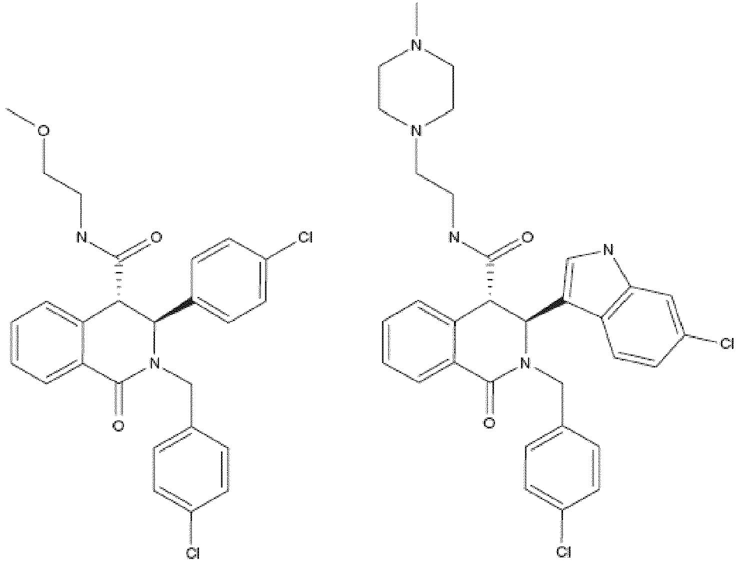

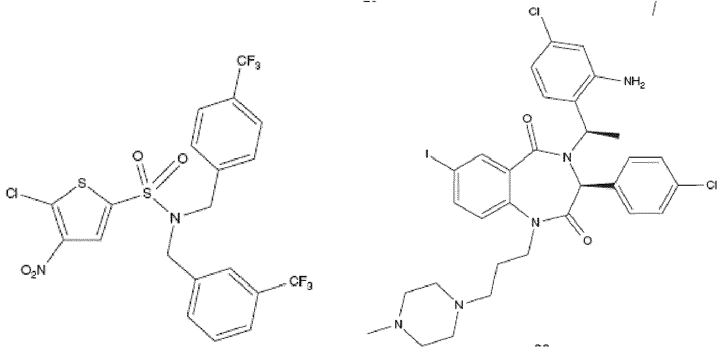

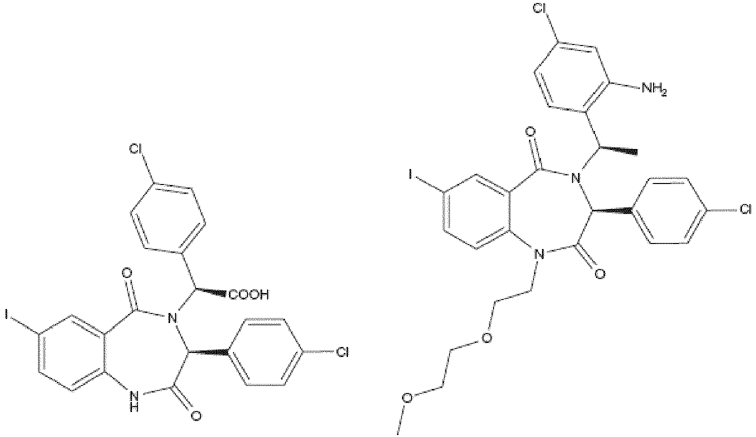

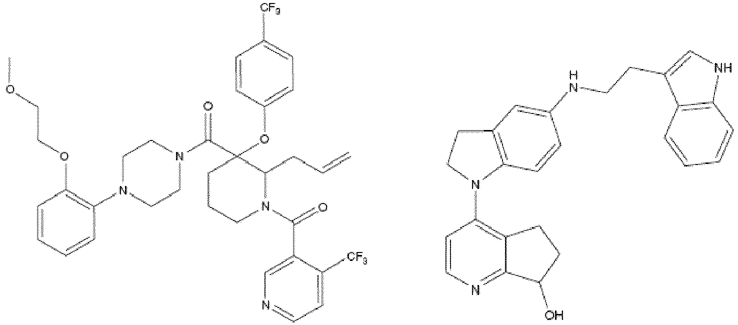

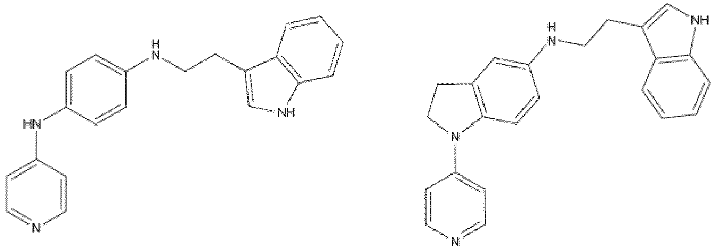

2. The method for producing an influenza virus as claimed in claim 1, **characterized in that** the Mdm2 antagonist is used for quantitatively improving the production of the influenza virus produced.

3. The method for producing an influenza virus as claimed in claim 1 or 2, **characterized in that** the influenza virus is chosen from the subtypes A, B and C, and in particular the influenza subtype A viruses corresponding to the H1N1, H2N2, H3N2, H4N2, H4N6, H5N1, H5N2, H7N7 and H9N2 strains, and in particular the A H3N2 virus.

4. The method for producing an influenza virus as claimed in one of claims 1 to 3, **characterized in that** the production is carried out in a MDCK cell line, which is infected with an influenza virus, and **in that** the Mdm2 antagonist is added before, after or at the same time as the infection.

5. The method for producing an influenza virus as claimed in one of the preceding claims, comprising the following steps: a) infection with an influenza virus, and in particular with influenza vaccine seeds, of cells of the MDCK cell line in a cell culture medium; b) incubation of the production MDCK cell line selected and infected in step a) under conditions which allow the replication of said influenza virus, and in particular of the influenza vaccine seeds; c) harvesting of the whole influenza virus, and in particular of the vaccine seeds, produced in the culture supernatant and/or in said cells in culture.

6. The method for producing an influenza virus as claimed in claim 5, **characterized in that** it comprises the inactivation of the virus harvested in step c).

7. The method for producing an influenza virus as claimed in one of the preceding claims, **characterized in that** the virus is a wild-type virus, a primary viral isolate obtained from an infected individual, a recombinant virus, an attenuated virus, a reassorted virus, or a virus produced by reverse genetics.

8. A method for preparing a vaccine against an influenza virus, **characterized in that** it comprises the production of an influenza virus by means of a method as claimed in one of claims 1 to 7.

9. The method for preparing a vaccine as claimed in claim 8, **characterized in that** it comprises the production of antigenic surface proteins using a whole influenza virus produced according to the method of one of claims 1 to 7.

10. The method as claimed in claim 8 or 9, **characterized in that** the vaccine obtained corresponds to killed pathogens or to live attenuated pathogens.
